# EUROPEAN PATENT APPLICATION

(11) **EP 3 639 842 A2**
(43) Date of publication of application: **22.04.2020**
(21) Application number: 18818565.6
(22) Date of filing: 28.05.2018
(51) Int. Cl.: A61K 38/16, A61K 9/00, A61K 9/08

(54) **PHARMACEUTICAL COMPOSITION CONTAINING GLY-THYMOSIN 4 (GLY-T 4) FOR TREATMENT OF DRY EYE**

(30) Priority: 14.06.2017 KR 20170074962
(71) Applicant: Huons Co., Ltd., Seongnam-si, Gyeonggi-do 13486 (KR)
(72) Inventor: KIM, Young Mok, Seoul 04732 (KR); KIM, Wanseop Paul, Yongin-si, Gyeonggi-do 16822 (KR); UM, Key An, Suwon-si, Gyeonggi-do 16295 (KR)
(74) Representative: Dehns
(86) International application number: PCT/KR2018/006030
(87) International publication number: WO 2018/230858

(57) **Abstract**

The present invention relates to a pharmaceutical composition for treating dry eye syndrome, which comprises Gly-Tβ4 as an active ingredient, and the pharmaceutical composition restores damaged corneal epithelial cells and also has an effect of proliferating goblet cells, and thus is effective for treating, preventing, or alleviating dry eye syndrome.

## Description

### [Technical Field]

The present invention relates to a composition for preventing or treating dry eye syndrome, which comprises Gly-thymosin β4 (Gly-Tβ4) as an active ingredient.

### [Background Art]

Dry eye syndrome refers to abnormalities in the tear layer due to reduced or fluctuating quantity and quality of the tear layer that moisturizes the eyes and maintains a soft and comfortable eye condition, and includes not only irritating symptoms such as irritation, a sense of a foreign body, dryness, or the like, which results from lack of tears, excessive evaporation of tears, or damage to the eye surface due to unbalanced tear components, but also keratitis, membrane epithelial wounds, or the like resulting from these symptoms. Representative examples of dry eye syndrome include, but are not limited to, xerosis conjunctiva and keratoconjunctivitis sicca.

Such dry eye syndrome is known to occur in the following cases of: i) evaporation of tears from the surface of the eye; ii) non-secretion of tears due to inflammation of the tear glands; and iii) a lack of tears resulting from the fact that due to damage to the mucin layer, the water layer thereon is unable to sufficiently cover the surface of the eye, but the exact causes thereof have not yet been found.

As therapeutic agents for the treatment of dry eye syndrome, RESTASIS® (Cyclosporin A) by Allergan, DIQUAS® (Diquafosol) by Santen, Hyalein® (Sodium Hyaluronate) by Santen and Generics, and the like are commercially available, but these therapeutic agents have drawbacks, such as a sensation of burning eyes and discomfort in applying eye drops and are not suitable for use in multifactorial dry eye syndrome. In addition, such therapeutic agents exhibit an anti-inflammatory action or an effect of improving dry eye syndrome through the promotion of mucin secretion, but the fundamental treatment thereof is difficult.

Meanwhile, Gly-thymosin β4 (Gly-Tβ4) is a peptide consisting of a 44 amino acid sequence, which is produced by adding glycine (Gly) to the N-terminus of conventional Thymosin β4 (Tβ4).

Gly-Tβ4 has been discovered through the already known quaternary structural analysis of Tβ-4 and the genetic engineering modification of the N-terminus of the protein and inherently has activity different from Tβ-4.

Tβ4 is one of the major thymic factors whose structure and function are relatively defined and known. Tβ4 is the first polypeptide isolated from thymosin fraction 5 (TF5) extracted from a bovine mammary gland and is known to have 43 amino acids and a molecular weight of 4.963 KD without disulfide bonds and glycosylation (The Journal of Biological Chemistry, Vol. 257, No.2, pp.1000-1006, 1982, Chemical Characterization of Thymosin beta). Tβ4 is a protein expressed during the development of heart disease in embryos and is known to promote the migration of cardiac cells and affect the survival of these cells (Thymosin β4 activates integrin-linked kinases and enhances cardiac cell migration, survival, and heart repair, Nature, Vol. 432, Nov. 25, 2004).

As a result of having conducted research on Gly-Tβ4, the inventors of the present invention confirmed that symptoms of patients with dry eye syndrome were alleviated through dry eye syndrome animal models and clinical studies, and thus completed the present invention.

### [Disclosure]

### [Technical Problem]

While having conducted research on Tβ4 and derivatives thereof, the inventors of the present invention confirmed that Gly-thymosin β4 (Gly-Tβ4) was more effective for treating dry eye syndrome than Tβ4, and a composition comprising Gly-Tβ4 as an active ingredient could be used as a therapeutic agent for dry eye syndrome, and thus completed the present invention.

### [Technical Solution]

To address the above-described problems, the present invention provides a pharmaceutical composition for treating, alleviating, or preventing dry eye syndrome, which comprises Gly-thymosin β4 (Gly-Tβ4) as an active ingredient.

The Gly-Tβ4 may comprise an amino acid sequence of SEQ ID NO: 1.

The content of the Gly-Tβ4 may range from 0.005% (w/v) to 5% (w/v) with respect to a total weight of the composition.

The pharmaceutical composition may be in an eye drop form.

The dry eye syndrome may be aqueous tear-deficient dry eye or evaporative dry eye.

The dry eye syndrome may be selected from the group consisting of alacrima, xerophthalmia, Sjogren syndrome, keratoconjunctivitis sicca, Stevens-Johnson syndrome, ocular pemphigoid, dry eye after an ophthalmic operation, dry eye accompanied by allergic conjunctivitis, and dry eye-like conditions comprising tear decrement of visual display terminal (VDT) workers and tear decrement without any systemic symptom caused by a dry room due to an air conditioner. The pharmaceutical composition may restore damaged corneal epithelial cells and may also proliferate goblet cells.

The present invention also provides an artificial tear drop for alleviating or preventing dry eye syndrome, which comprises Gly-thymosin β4 (Gly-Tβ4) as an active ingredient.

The present invention provides a method of preventing or treating dry eye syndrome, which comprises administering, to a subject, a pharmaceutical composition comprising Gly-thymosin β4 (Gly-Tβ4) as an active ingredient.

The present invention also provides a use of a pharmaceutical composition for preventing or treating dry eye syndrome, the pharmaceutical composition comprising Gly-thymosin β4 (Gly-Tβ4) as an active ingredient.

### [Advantageous Effects]

The present invention relates to a pharmaceutical composition for treating dry eye syndrome, which comprises Gly-Tβ4 as an active ingredient, and the pharmaceutical composition restores damaged corneal epithelial cells and also has an effect of proliferating goblet cells, and thus is effective for treating, preventing, or alleviating dry eye syndrome.

### [Description of Drawings]

FIG. 1 illustrates the results of measuring tear volumes of experimental groups (Group 1: EDE, Group 2: Vehicle, Group 3: Gly-Tβ4 10 µg/mL, Group 4: Gly-Tβ4 100 µg/mL, and Group 5: Gly-Tβ4 1,000 µg/mL).
FIG. 2 illustrates the results of measuring the tear film break-up time of experimental groups (Group1: EDE, Group 2: Vehicle, Group 3: Gly-Tβ4 10 µg/mL, Group 4: Gly-Tβ4 100 µg/mL, and Group 5: Gly-Tβ4 1,000 µg/mL).
FIG. 3 illustrates the results of quantifying wounds in the cornea through fluorescent staining of experimental groups (Group 1: EDE, Group 2: Vehicle, Group 3: Gly-Tβ4 10 µg/mL, Group 4: Gly-Tβ4 100 µg/mL, Group 5: Gly-Tβ4 1,000 µg/mL).
FIG. 4 illustrates flow cytometric results for measuring the distribution of T cells in the conjunctiva.
FIG. 5 illustrates flow cytometric results for measuring the distribution of T cells in the lacrimal glands.
FIG. 6 illustrates the results of measuring the goblet cell density of experimental groups (Group 1: EDE, Group 2: Vehicle, Group 3: Gly-Tβ4 10 µg/mL, Group 4: Gly-Tβ4 100 µg/mL, Group 5: Gly-Tβ4 1,000 µg/mL).
FIG. 7 illustrates fluorescent staining results for confirming damaged corneal epithelial cells in experimental groups (Group 1: EDE, Group 2: Vehicle, Group 3: Gly-Tβ4 10 µg/mL, Group 4: Gly-Tβ4 100 µg/mL, Group 5: Gly-Tβ4 1,000 µg/mL).
FIG. 8 illustrates the results of comparing the concentrations of regenerated goblet cells for the comparison in efficacy between Gly-Tβ4 and Tβ4.

### [Best Mode]

Gly-thymosin β4 (Gly-Tβ4) is a Tβ4 derivative (amino acid sequence of SEQ ID NO: 1) consisting of a 44 amino acid sequence, which is produced by adding glycine (Gly) to the N-terminus of conventional Thymosin β4 (Tβ4). Gly-Tβ4 may be expressed through a genetic recombination method, or may be prepared by chemical synthesis, but without being limited thereto, may be prepared using known methods. More preferably, Gly-Tβ4 may be obtained through recombination and expression methods through genetic recombination, and more particularly, *E. coil* transformed with a plasmid carrying the Gly-Tβ4 gene may be cultured to be overexpressed in the form of a recombinant protein (GST-Tβ4 fusion protein), followed by purification and treatment with thrombin, thereby obtaining Gly-Tβ4 as a final product.

Gly-Tβ4 may promote the migration of corneal epithelial cells, thereby promoting the formation of collagen and laminin-5. Such collagen and laminin-5 may restore corneal damage and finally improve dry eye. In addition, without being limited thereto, Gly-Tβ4 is known to have the effects of promoting MUC5AC (goblet cells present on the ocular surface) and goblet cell growth to increase mucin and helping the regeneration of damaged corneal epithelial cells to cure dry eye.

Dry eye syndrome comprises tear film disorders due to tear deficiency or excessive evaporation of tears and may also comprise symptoms of eye discomfort that causes damage to the interpalpebral eye surface. In addition, dry eye syndrome may refer to a multifactorial disease of the tears and ocular surface that results in symptoms of discomfort, visual impairment, and tear film instability with potential damage to the ocular surface, and such disease may be accompanied by increased osmolarity of the tear film and inflammation of the ocular surface.

Dry eye syndrome comprises aqueous tear-deficient dry eye and evaporative dry eye, and the aqueous tear-deficient dry eye may comprise diseases caused by a failure of lacrimal gland tear secretion. In addition, evaporative dry eye may comprise diseases caused by excessive loss of moisture from the exposed eye surface in the presence of normal lacrimal gland secretory function.

The present invention relates to a pharmaceutical composition for treating, alleviating, or preventing dry eye syndrome, which comprises Gly-thymosin β4 (Gly-Tβ4) as an active ingredient.

The pharmaceutical composition may be an ophthalmic preparation.

The preparation may be in the form of a gel, a lotion, an aerosol, a cream, a suspension, a solution, a dispersion, or oil. The composition may comprise pharmaceutically acceptable, more preferably, ophthalmically acceptable excipients or carriers. The term "pharmaceutically acceptable" generally means a use for animals, particularly mammals, especially humans.

In addition, the composition may comprise a stabilizer, a solubilizer, buffer, a preservative, a thickening agent, or other excipients.

In the present invention, the carrier may be any carrier suitable for topical administration or general-purpose administration. More specifically, the carrier may be water, a mixture of water and water-miscible solvents [Examples of the water-miscible solvents comprise C₁-C₇ alkanols, vegetable oils or mineral oils (e.g., 0.5 wt% to 5 wt% of hydroxyethylcellulose, ethyl oleate, carboxymethyl cellulose, polyvinyl-pyrrolidone, and the like), acrylates (e.g., salts of polyacrylic acids or ethyl acrylate), methacrylates, polyacrylamides, natural products (e.g., gelatin, alginates, pectin, tragacanth, karaya gum, xanthan gum, carrageenan, agar, Acacia gum, starch derivatives (e.g., starch acetate and hydroxypropyl starch))], other non-toxic water-soluble polymers for ophthalmic purposes (e.g., cellulose derivatives (e.g., methylcellulose, alkali metal salts of carboxymethylcellulose, hydroxymethylcellulose, hydroxyethylcellulose, methylhydroxypropylcellulose, and hydroxypropylcellulose)), other synthetic products (e.g., polyvinyl alcohol, polyvinylpyrrolidone, polyvinyl methyl ether, polyethylene oxide, crosslinked polyacrylic acids (e.g., neutral carbopol)), or mixtures of these polymers.

The buffer may be selected from the group consisting of a borate-based buffer, a hydrogen carbonate/carbonate-based buffer, a gluconate-based buffer, a phosphate-based buffer, a propionate-based buffer, and a tromethamine (TRIS)-based buffer. Specifically, the buffer may be a tromethamine-based buffer or a borate-based buffer. The amount of a buffer substrate may be, for example, an amount needed to ensure and maintain a physiologically acceptable pH range. The physiologically acceptable pH range may be between pH 5 and pH 9, particularly between pH 6 and pH 8.2, and more particularly between pH 6.8 and pH 8.1.

The preservative may be quaternary ammonium salts (e.g., cetrimide, benzalkonium chloride, or benzoxonium chloride), alkyl-mercury salts of thiosalicylic acid (e.g., thimerosal, phenylmercuric acid nitrate, phenylmercuric acid acetate, or phenylmercuric acid borate), parabens (e.g., phenylparaben or propylparaben), alcohols (e.g., chlorobutanol, benzyl alcohol, or phenyl ethanol), guanidine derivatives (e.g., chlorohexidine or polyhexamethylene biguanide), or sorbic acid. Specifically, the preservative may be a quaternary ammonium salt or a paraben. The amount of the preservative is not particularly limited as long as it is an amount that can prevent secondary contamination caused by bacteria and fungi during use.

To appropriately maintain the viscosity of the ophthalmic preparation, the ophthalmic preparation may comprise, without being limited thereto, the following formulations: (a) monomeric polyols (e.g., tyloxapol, glycerol, propylene glycol, and ethylene glycol); (b) polymeric polyols (e.g., polyethylene glycol (e.g., PEG 300 and PEG 400)); (c) cellulose derivatives (cellulose-based polymers) (e.g., hydroxyethylcellulose, hypromellose, hydroxypropylmethyl cellulose, methylcellulose, carboxymethylcellulose sodium, and hydroxylpropylcellulose); (d) dextrans (e.g., dextran 70); (e) water-soluble proteins (e.g., gelatin); (f) vinyl polymers (e.g., polyvinyl alcohol and polyvinyl pyrrolidone); (g) other polyols (e.g., polysorbate 80 and povidone); (h) carbomers (e.g., carbomer 934P, carbomer 941, carbomer 940, and carbomer 974P); and (i) polysaccharides/glycosaminoglycans (e.g., hyaluronan (hyaluronic acid/hyaluronate), chondroitin sulfate).

The present invention provides a method of preventing or treating dry eye syndrome, comprising administering the above-described composition to a subject.

The subject may comprise animals, more particularly mammals, and more preferably humans.

The administration may comprise transdermal administration, parenteral administration, intranasal (such as inhalation) administration, and administration via the mucous membrane, and more particularly, may comprise bringing the composition into direct or indirect contact with ocular tissue.

The composition comprises Gly-thymosin β4 (Gly-Tβ4) as an active ingredient and may be administered alone or in combination with other drugs.

The composition may comprise Gly-Tβ4 in an amount of 0.005% (w/v) to 5% (w/v), more preferably 0.01% (w/v) to 0.1% (w/v), with respect to the total amount of the composition.

The present invention provides an artificial tear drop for alleviating or preventing dry eye syndrome, which comprises Gly-thymosin β4 (Gly-Tβ4) as an active ingredient.

The present invention will be described in detail with reference to the following examples to aid in understanding thereof. However, examples according to the present invention may be modified in many different forms and the following examples are not intended to limit the scope of the present invention.

### [Mode of the Invention]

### Example 1. Preparation of Gly-Tβ4

For the preparation of HU024 (Gly-Tβ4), 1 vial of WCB is thawed and seed-cultured twice to multiply the number of cells, followed by culturing in a production bioreactor. After the culturing is completed, a slurry is prepared, and then cells are disrupted and subjected to depth/micro filtration (UF/DF). A culture supernatant is subjected to anion exchange chromatography, followed by affinity chromatography. Thereafter, enzymatic degradation proceeds, followed by anion exchange chromatography, cation exchange chromatography, and UF/DF, thereby completing the preparation of a final crude formulation.

The following table is a configuration for culturing and purification based on 500 L.

**[Table 1]**

| 500 L preparation process | |
|---|---|
| Culturing (fermentation) | Purification |
| Cell bank thaw (WCB) | Anion Exchange Chromatography |
| 2000 mL Erlenmeyer flask 1 ea (500 mL) | Affinity Chromatography |
| 25 L Seed Bioreactor (15 L) | Enzyme Digestion |
| 500 L Production Bioreactor (440 L) | Affinity Chromatography |
| Harvest | Cation Exchange chromatography |
| Slurry Preparation | Ultrafiltration/Diafiltration |
| Cell Disruption | Sterile Filtration |
| Depth Filtration/Micro Filtration | Storage of DS |

### Experimental Example 1. Experiment for Efficacy of Gly-Tβ4 on Treatment of Dry Eye Syndrome

The efficacy of Gly-Tβ4 (hereinafter referred to as HU024) was examined as follows using an experimental dry eye (EDE) model produced by administration of scopolamine (0.5 mg/0.2 ml, 3 times a day) and then air drafting of dry air in a cage via a fan.

### 1. Experimental Groups

**[Table 2]**

| Group | Description | Subject |
|---|---|---|
| G1 | EDE Control Group | N=5 |
| G2 | EDE+ Vehicle | N=5 |
| G3 | HU024 (Gly-Tβ4) 10 µg/ml | N=5 |
| G4 | HU024 (Gly-Tβ4) 100 µg/ml | N=5 |
| G5 | HU024 (Gly-Tβ4) 1,000 µg/ml | N=5 |

### 2. Administration Method

A single droplet of Gly-Tβ4 was administered to each of the eyes of each mouse three times a day.

### 3. Evaluation Method

1) As clinical parameters, a. tear volume (measured on day 7 and day 14), b. tear film BUT (measured on day 7 and day 14), and c. fluorescent staining (measured on day 7 and day 14) were used.
2) As a biochemical parameter, a. inflammatory cytokine analysis (target cytokines: IL-1β and TNF-α) was performed.
3) As immunological/cellular parameters, a. goblet cell density examination (using a PAS staining method) and b. inflammatory T cell distribution examination (flow cytometer, CD4+CCR5+) were performed.

### 4. Experimental Results

### 1.1 Tear Volume Measurement

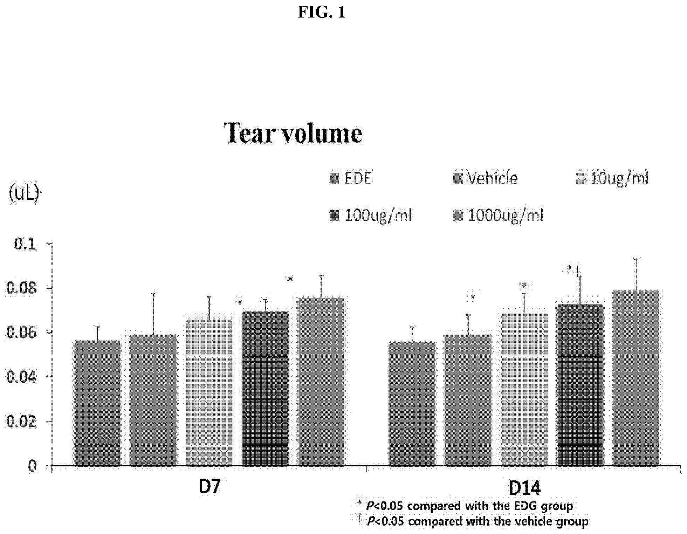

The secretion of tears was measured by bringing phenol-red impregnated cotton threads into contact with the conjunctival sac of the lateral canthus of each mouse for 20 seconds and measuring the lengths thereof using a SMZ microscopy, and then the lengths were converted into volumes through substitution into a given formula. The results are the same as shown below (see FIG. 1).

**[Table 3]**

| | Group 1 (EDE) | Group 2 (Vehicle) | Group 3 (10 µg/mL) | Group4 (100 µg/mL) | Group 5 (1,000 µg/mL) |
|---|---|---|---|---|---|
| Day 7 | 0.057 ± 0.006 | 0.059 ± 0.018 | 0.066 ± 0.011 | 0.069 ± 0.006 | 0.076 ± 0.010 |
| Day 14 | 0.056 ± 0.007 | 0.059 ± 0.009 | 0.069 ± 0.009 | 0.073 ± 0.013 | 0.079 ± 0.014 |

### 1.2 Tear Film BUT

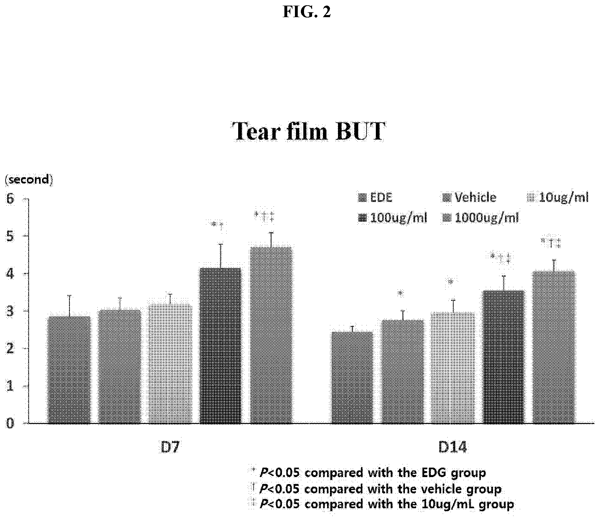

For the examination of tear film break-up time to determine tear film stability, which is the most important clinical factor in dry eye, 1 µl of a 1% fluorescent dye (fluorescein) was dropped into the lower conjunctival sac of each mouse, and then each mouse was allowed to blink their eyes about three times, the eyes were gently made open, and then the time (seconds) until damage first occurred in the stained tear film was measured using a slit lamp microscope equipped with a cobalt blue filter. This process was repeated three times to obtain an average of the measurement results. The results thereof are shown below (see FIG. 2).

**[Table 4]**

| | Group 1 (EDE) | Group 2 (Vehicle) | Group 3 (10 µg/mL) | Group 4 (100 µg/mL) | Group 5 (1,000 µg/mL) |
|---|---|---|---|---|---|
| Day 7 | 2.87±0.54 | 3.03±0.32 | 3.18±0.27 | 4.15±0.64 | 4.70±0.29 |
| Day 14 | 2.44±0.16 | 2.76±0.25 | 2.96±0.33 | 3.55±0.39 | 4.07±0.29 |

### 1.3 Fluorescent Staining

1 µL of a 1% fluorescent dye was dropped into the eyes of each mouse, followed by washing with saline, and then the cornea of each mouse was observed using a slit lamp microscope to perform evaluation through scoring of the degree of epithelial injury (the degree to which the fluorescent dye was smeared). To this end, the cornea was divided into four zones, and then each zone was evaluated from 0 to 4 points as follows and the points were summed up (a total of 0 to 16 points).
① Absent
② Slightly punctuate staining < 30 spots
③ Punctate staining > 30 spots, but not diffuse
④ Severe diffuse staining but no positive plaque

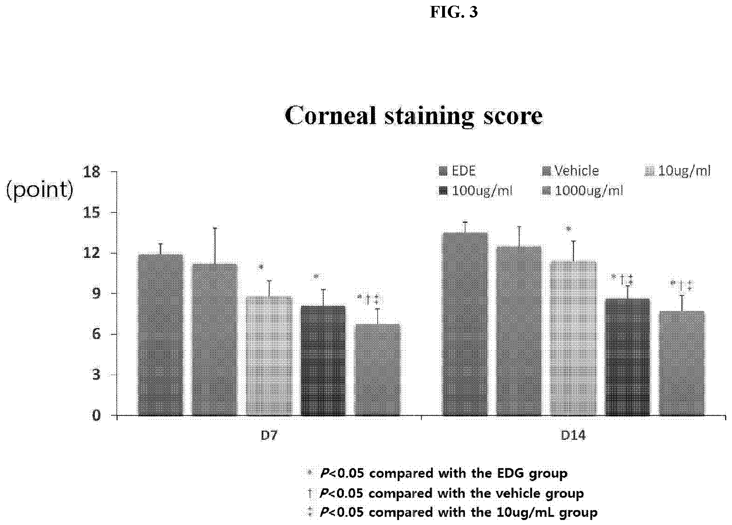

The results thereof are shown below (see FIG. 3)

**[Table 5]**

| | Group 1 (EDE) | Group 2 (Vehicle) | Group 3 (10 µg/mL) | Group 4 (100 µg/mL) | Group 5 (1,000 µg/mL) |
|---|---|---|---|---|---|
| Day 7 | 11.9±1.79 | 11.2±2.66 | 8.8±1.14 | 8.1±1.20 | 6.7±1.16 |
| Day 14 | 13.5±1.08 | 12.5±1.43 | 11.4±1.51 | 8.6±0.97 | 7.7±1.16 |

### 2.1 Biochemical Parameter

Six conjunctival tissues were incised and removed from both eyes of three mice per each group, and then were immersed in a protease inhibitor-containing lysis buffer for 30 minutes, followed by centrifugation and storage at -70 °C. Subsequently, the concentrations (pg/ml) of inflammatory cytokines (IL-1β and TNF-α) were measured using a Luminex multiplex immunobead assay, and the results thereof are shown below.

**[Table 6]**

| | Conjunctival Gland | | | | |
|---|---|---|---|---|---|
| | Group 0 (EDE) | Group 1 (Vehicle) | Group 2 (10 µg/mL) | Group 3 (100 µg/mL) | Group 4 (1,000 µg/mL) |
| IL-1β | 21.22 | 9.13 | 12.52 | 3.67 | 3.13 |
| TNF-α | 20.02 | 18.88 | 15.86 | 11.15 | 10.22 |

**[Table 7]**

| | Conjunctival Gland | | | | |
|---|---|---|---|---|---|
| | Group 0 (EDE) | Group 1 (Vehicle) | Group 2 (10 µg/mL) | Group 3 (100 µg/mL) | Group 4 (1,000 µg/mL) |
| IL-1β | 21.22 | 9.13 | 12.52 | 3.67 | 3.13 |
| TNF-α | 20.02 | 18.88 | 15.86 | 11.15 | 10.22 |

### 3.1 Immunological/Cellular Parameter

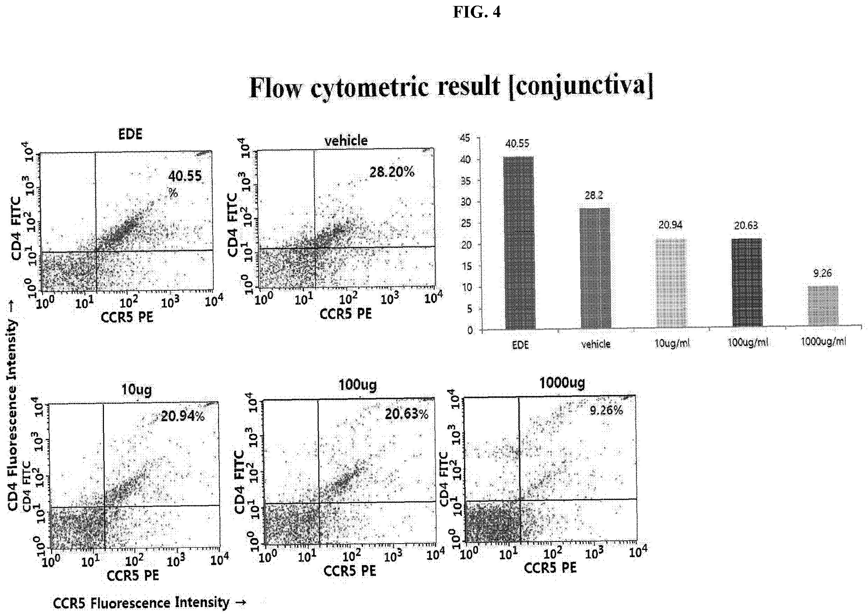

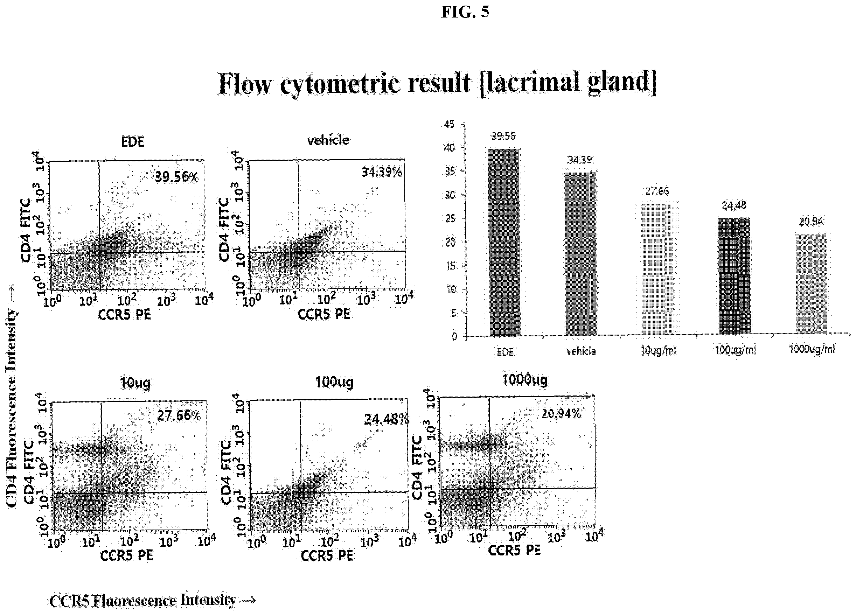

The conjunctiva was excised and sectioned with scissors, and then mixed with 0.5 mg/mL collagenase type D at 37 °C for 60 minutes. The tissue was ground and then allowed to pass through a cell strainer, followed by centrifugation. Flow cytometry was performed using a fluorescein-conjugated anti-CD4 antibody and a phycoerythrin-conjugated anti-IFN-y antibody to calculate the number of CD4⁺CCR5⁺T cells. The results of measuring the distribution of T cells are shown below (see FIGS. 4 and 5).

**[Table 8]**

| | Group 1 (EDE) | Group 2 (Vehicle) | Group 3 (10 µg/mL) | Group 4 (100 µg/mL) | Group 5 (1,000 µg/mL) |
|---|---|---|---|---|---|
| Conjunctiva | 40.55 | 28.2 | 20.94 | 20.63 | 9.26 |
| Lacrimal gland | 39.56 | 34.39 | 27.66 | 24.48 | 20.94 |

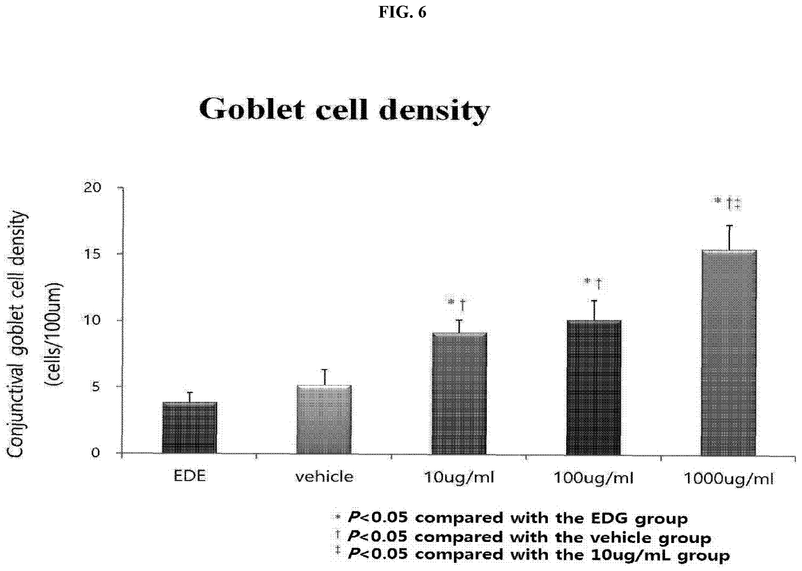

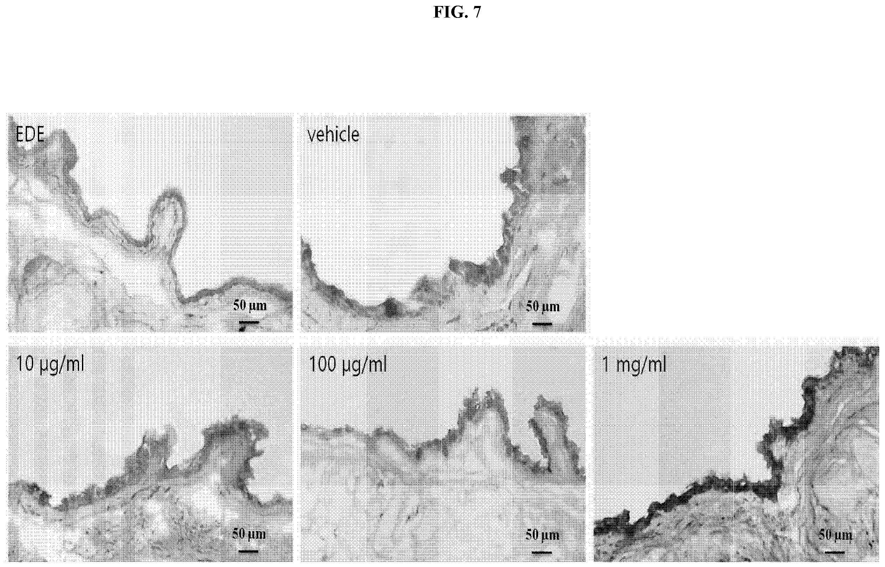

Periodic acid-Schiff staining was performed to evaluate the density of goblet cells. The eyes and appendages of mice were excised, fixed in 4% paraformaldehyde, embedded in paraffin, and then subjected to PAS staining for tissue sections. In each section, the density of goblet cells in the conjunctiva was calculated using a microscope and image-analysis software. As a result, the density of goblet cells is shown below (see FIGS. 6 and 7).

**[Table 9]**

| Group 1 (EDE) | Group 2 (Vehicle) | Group 3 (10 µg/mL) | Group 4 (100 µg/mL) | Group 5 (1,000 µg/mL) |
|---|---|---|---|---|
| 3.8 ± 0.8 | 5.2 ± 1.2 | 9.2 ± 1.0 | 10.2 ± 1.5 | 15.5 ± 1.9 |

It was confirmed that the tear volume was increased in dry eye in a manner dependent on the concentration of HU024, and the tear film BUT was also increased in a manner dependent on the concentration of HU024.

In addition, in the case of fluorescent staining, it was confirmed that, as dry eye syndrome became more severe, the number of stained spots of damaged corneal epithelial cells was increased, and it can be seen that this value was decreased.

Through these three methods, the concentration-dependent increase in tear volume, the increase in tear film BUT, and the decrease in fluorescent staining were confirmed, from which it was confirmed that dry eye symptoms were alleviated or treated in a dry eye model.

It was also confirmed that the amounts of interleukin-lbeta and TNF-alpha, which have inflammatory activity, were decreased in a concentration-dependent manner, from which the anti-inflammatory activity of HU024 could be confirmed. It was also confirmed that the number of CD4+CCR5+ T cells was decreased in a concentration-dependent manner even in the flow cytometric results, from which it could be indirectly confirmed that inflammation was alleviated due to a decrease in the amount of T cells specifically present in inflammatory sites.

In addition, it can be seen through PAS staining that goblet cell density is increased in a manner dependent on the concentration of HU024, and as can be seen from the previous fluorescent staining results, goblet cells were proliferated as the number of the damaged corneal epithelial cells was decreased.

As can be seen from these results, it was confirmed that HU024 exhibited an anti-inflammatory effect and a cell regeneration effect.

### Comparative Example. Experiment for Efficacy Comparison (Experiment for Comparison in Efficacy between Gly-Tβ4 and Tβ4)

Experimental groups were configured as follows.
Group 1: EDE, Group 2: Vehicle, Group 3: WT-Tβ4, Group 4: Gly-Tβ4

Through an increase in goblet cell density values, it can be confirmed that a decreased number of goblet cells in the cornea due to experimental induction of dry eye is increased due to regeneration thereof by a drug.

**[Table 10]**

| | Group 1 (EDE) | Group 2 (Vehicle) | Group 3 WT-Tβ4 | Group 4 Gly-Tβ4 |
|---|---|---|---|---|
| Goblet Cell Density | 3.3 ± 0.8 | 5.0 ± 1.3 | 9.7 ± 0.8 | 11.0 ± 0.8 |

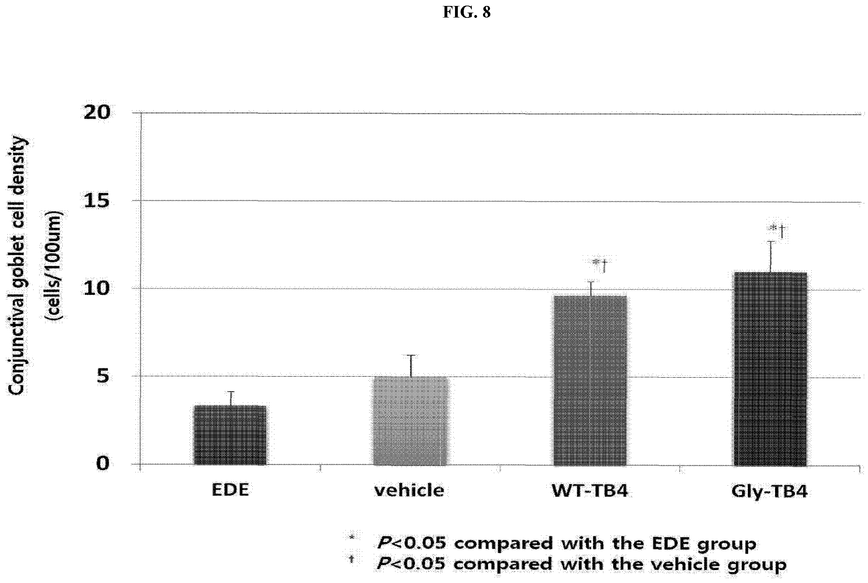

As can be seen from the above table, it can be confirmed that a greater number of goblet cells is regenerated in the case of Group 4: Gly-Tβ4 compared to the case of Group 3: WT-Tβ4 (see FIG. 8).

### [Industrial Applicability]

The present invention relates to a pharmaceutical composition for treating dry eye syndrome, which comprises Gly-Tβ4 as an active ingredient, and the pharmaceutical composition restores damaged corneal epithelial cells and also has an effect of proliferating goblet cells, and thus is effective for the treatment, prevention, or alleviation of dry eye syndrome and can be usefully used in the medical industry for the development of a therapeutic agent for dry eye syndrome.

## Claims

1. A pharmaceutical composition for treating, alleviating, or preventing dry eye syndrome, the pharmaceutical composition comprising Gly-thymosin β4 (Gly-Tβ4) as an active ingredient.

2. The pharmaceutical composition of claim 1, wherein the Gly-Tβ4 consists of an amino acid sequence of SEQ ID NO: 1.

3. The pharmaceutical composition of claim 1, wherein a content of the Gly-Tβ4 ranges from 0.005% (w/v) to 5% (w/v) with respect to a total volume of the composition.

4. The pharmaceutical composition of claim 1, wherein the pharmaceutical composition is in an eye drop form.

5. The pharmaceutical composition of claim 1, wherein the dry eye syndrome is aqueous tear-deficient dry eye or evaporative dry eye.

6. The pharmaceutical composition of claim 1, wherein the dry eye syndrome is selected from the group consisting of alacrima, xerophthalmia, Sjogren syndrome, keratoconjunctivitis sicca, Stevens-Johnson syndrome, ocular pemphigoid, dry eye after an ophthalmic operation, dry eye accompanied by allergic conjunctivitis, and dry eye-like conditions comprising tear decrement of visual display terminal (VDT) workers and tear decrement without any systemic symptom caused by a dry room due to an air conditioner.

7. The pharmaceutical composition of claim 1, wherein the pharmaceutical composition restores damaged corneal epithelial cells and proliferates goblet cells.

8. An artificial tear drop for alleviating or preventing dry eye syndrome, the artificial tear drop comprising Gly-thymosin β4 (Gly-Tβ4) as an active ingredient.

9. A method of preventing or treating dry eye syndrome, the method comprising administering, to a subject, a pharmaceutical composition comprising Gly-thymosin β4 (Gly-Tβ4) as an active ingredient.

10. A use of a pharmaceutical composition for preventing or treating dry eye syndrome, the pharmaceutical composition comprising Gly-thymosin β4 (Gly-Tβ4) as an active ingredient.


<110>    Huons

<120>    PHARMACEUTICAL COMPOSITION FOR TREATMENT OF DRY EYE HAVING
         Gly-Tb4

<130>    MPCT18-032

<150>    KR 10-2017-0074962
<151>    2017-06-14

<160>    1

<170>    KopatentIn 2.0

<210>    1
<211>    44
<212>    PRT
<213>    recombinant Gly-Thymosin beta4

<400>    1
Gly Ser Asp Lys Pro Asp Met Ala Glu Ile Glu Lys Phe Asp Lys Ser
  1               5                  10                  15

Lys Leu Lys Lys Thr Glu Thr Gln Glu Lys Asn Pro Leu Pro Ser Lys
             20                  25                  30

Glu Thr Ile Glu Gln Glu Lys Gln Ala Gly Glu Ser
         35                  40